# EUROPÄISCHE PATENTSCHRIFT

(11) **EP 0 967 958 B1**
(45) Veröffentlichungstag und Bekanntmachung des Hinweises auf die Patenterteilung: **14.02.2007**
(21) Anmeldenummer: 98913622.1
(22) Anmeldetag: 03.03.1998
(51) Int. Cl.: A61K 8/14, A61K 8/60, A61Q 5/00, A61Q 19/00, A61Q 19/10, A61Q 19/08

(54) **KÖRPERREINIGUNGSMITTEL**
BODY CLEANSING AGENT
NETTOYANT CORPOREL

(30) Priorität: 12.03.1997 DE 19710149
(43) Veröffentlichungstag der Anmeldung: 05.01.2000
(73) Patentinhaber: Henkel Kommanditgesellschaft auf Aktien, 40589 Düsseldorf (DE)
(72) Erfinder: FÖRSTER, Thomas, D-40699 Erkrath (DE); HOLLENBROCK, Martina, D-40599 Düsseldorf (DE); SCHOLZ, Wolfhard, D-47829 Krefeld (DE); PITTERMANN, Wolfgang, D-40625 Düsseldorf (DE); SCHMITT, Michael, D-42781 Haan (DE)
(86) Internationale Anmeldenummer: PCT/EP1998/001174
(87) Internationale Veröffentlichungsnummer: WO 1998/040044

(56) Entgegenhaltungen:
- EP-A- 0 641 557
- EP-A- 0 705 593
- FR-A- 2 666 015

## Beschreibung

Die Erfindung betrifft Körperreinigungsmittel auf Basis von wäßrigen Zubereitungen wasserlöslicher Tenside, in die lipidlösliche, kosmetische oder pharmazeutische Wirkstoffe in einer Weise eingearbeitet sind, die eine erhöhte Aufnahme der Wirkstoffe in die Haut während des Reinigungsvorganges bewirkt.

Es ist bekannt, Körperreinigungsmitteln kosmetische oder dermatologische Wirkstoffe zuzusetzen, um gleichzeitig mit dem Reinigungsvorgang eine dermatologisch günstige oder kosmetisch erwünschte Beeinflussung des Hautzustandes zu erreichen.

Für diesen Zweck eignen sich jedoch bisher nur wasserlösliche Wirkstoffe, da nur diese in der relativ kurzen Zeit des Waschvorgangs ausreichend mit der Haut in Kontakt treten, um dort eine Wirkung zu entfalten. Man hat zwar versucht, auch öllösliche Wirkstoffe durch Emulgierung oder Solubilisation homogen in wäßrige Reinigungsmittel einzuarbeiten, die dabei erhaltenen Wirkungen auf die Haut konnten jedoch nicht befriedigen, da die Wirkstoffe entweder in Emulsionströpfchen eingeschlossen oder in Tensidmizellen solubilisiert waren.

Die EP-A-0 705 593 und die EP-A-0 641 557 offenbaren ÖL-in-Wasser-Emulsionen, in welchen die Wirkstoff-Lipid-Tröpfchen mit Hilfe von Flüssigkristallen sind.

Es wurde daher nach einer Möglichkeit gesucht, lipidlösliche Wirkstoffe homogen und stabil in wäßrige Reinigungsmittel einzuarbeiten und dabei eine verbesserte Penetration der Wirkstoffe während der relativ kurzen Kontaktzeit mit der Haut zu erreichen. Als Ergebnis wurde gefunden, daß eine verbesserte Hautpenetration erreicht werden kann, wenn die Wirkstoffe als Komponente in Lipid-Tensid-Mischmizellen einer mittleren Teilchengröße unter 500 nm enthalten sind.

Gegenstand der Erfindung ist daher ein Verfahren zur Herstellung eines Körperreinigungsmittels in Form einer wässrigen Zubereitung mit einem Gehalt von wenigstens 5 Gew.-% wasserlöslicher ionischer oder nichtionischer Tenside und wenigstens 0,05 Gew.-% eines lipidlöslichen wasserunlöslichen kosmetischen oder dermatologischen Wirkstoffes, der mit einem polaren Lipid in Lipid-Tensid-Mischmizellen oder Flüssigkristallen solubilisiert ist, wobei die Emulsionsteilchen im wesentlichen einen Durchmesser von weniger als 500 nm aufweisen, wonach ein Konzentrat des in Lipid-Tensid-Mischmizellen oder FlüssiEkristallen solubilisierten lipidlöslichen, wasserunlöslichen kosmetischen oder dermatologischen Wirkstoffs mit einem Gehalt von 1 - 10 Gew.-% des Wirkstoffs hergestellt wird indem ein Gemisch aus dem Wirkstoff, einem polaren Lipid, das ausgewählt ist aus einem linearen C₁₂-C₂₂-Fettalkohol, einem linearen C₁₂-C₂₂-Alkandiol, einem C₈-C₂₂-Fettsäurepattialester eines C₃-C₈-Polyols mit 2 - 6 Hydroxylgruppen oder einem C₈-C₂₂-Fettsäuremonoalkanolamid eines C₂- oder C₃-Alkanolamins, und einem wasserlöslichen Tensid als Dispergator, das ausgewählt ist aus Alkylglycosiden bzw. Alkyl-(oligo)-glucosiden, mit Wasser auf eine Temperatur oberhalb des Schmelzpunktes des Lipids erwärmt und vermischt wird, wobei das Gewichtsverhältnis von polarem Lipid zu lipidlöslichem Wirkstoff 10 : 0.2 bis 10 : 2 und das Gewichtsverhältnis von Lipid zu Dispergator 1 : 2 bis 2 : 1 beträgt, so dass eine lamellare Flüssigkristalldispersion bzw. unterhalb der Schmelztemperatur eine lamellare Gelphase gebildet wird, die bei Verdünnung mit Wasser Lipid-Tensid-Mischzellen ausbildet, und mit einer wässrigen Tensidzubereitung vermischt wird.

Körperreinigungsmittel im Sinne der Erfindung sind flüssige oder pastöse wässrige Zubereitungen wie z.B. flüssige Seifen, Waschlotionen, Duschbadepräparate, Schaumbademittel oder Haarshampoos, die zur Reinigung der Haut oder der Kopfhaut und Haare aufgetragen und mit Wasser wieder von der Haut abgespült werden.

Wasserlösliche Tenside im Sinne der Erfindung sind solche ionischen oder nichtionischen Tenside, die bei 20°C zu wenigstens 5 Gew.-% klar im Wasser löslich sind. Bevorzugt geeignet sind z.B. schaumstarke anionische Sulfat- oder Sulfonattenside, ampholytische, zwitterionische, nichtionische Tenside oder Gemische davon. Beispiele solcher schaumstarker Aniontenside sind z.B. ein C₁₂-C₁₆-Alkylsulfate, z.B. in Form ihrer Alkanolaminsalze, die C₁₂-C₁₆-Alkylpolyglycolethersulfate, die Acylisethionate, die Acylsarkoside, die Fettsäuremonoglyceridsuifale, z.B. in Form ihrer Alkali-, Ammonium- oder Magnesium-Salze.

Geeignete zwitterionische Tenside sind vor allem die Betaintenside, z.B. das C₁₂-C₁₈-Alkyl-dimethyl-acetobetain, das Kokosacylamidopropyl-dimethylacetobetain oder Imidazoliniumbetaine und Sulfobetaine.

Geeignete schaumstarke Tenside sind vor allem die Gemische aus den genannten anionischen Sulfat- und Sulfonattensiden und zwitterionischen Betaintensiden. Auch Gemische aus anionischen Tensiden und nichtionischen Alkylglycosid-Tensiden sind als besonders schaumstark bekannt.

Geeignete nichtionische Tenside sind neben den genannten Alkylglycosiden bzw. Alkyl-(oligo)-glucosiden die Methylglucosid-Fettsäureester und deren Ethylenoxid-Addukte. Schließlich eignen sich auch Anlagerungsprodukte von Ethylenoxid an Fettalkohole, an Fettsäuren, an Fettsäuremonoglyceride, an Sorbitanfettsäureester, an Alkylglucoside, an Fettsäurealkanolamide und andere Fettstoffe mit Hydroxyl- oder Carboxylgruppen als nichtionische Tenside, wenn sie eine ausreichende Wasserlöslichkeit aufweisen. Dies ist in der Regel gegeben, wenn der Gehalt der lipophilen Acyl- oder Alkylgruppen im Molekül weniger als 50 Gew.-% ausmacht.

Die erfindungsgemäßen Körperreinigungsmittel enthalten solche wasserlöslichen Tenside bevorzugt in einer Menge von 5 - 30 Gew.-% und bevorzugt 0,1 - 1 Gew.-% eines lipidlöslichen Vitamins als Wirkstoff.

Als lipidlösliche kosmetische oder pharmazeutische Wirkstoffe im Sinne der Erfindung sind vor allem dermatologisch wirksame Verbindungen zu verstehen, die z.B. eine entzündungshemmende, lokalanaesthetische, hautweichmachende, antimikrobielle, strahlungsabsorbierende, hautschützende, durchblutungsfördernde oder vor Hautalterung schützende Wirkung haben und in Wasser kaum, wohl aber in z.B. Paraffinöl in einer Menge von mehr als 1 Gew.-% löslich sind.

Bevorzugt geeignete lipidlösliche Wirkstoffe sind z.B. Vitamine, wie Vitamin A (Retinole), Vitamin E (Tocopherole), Vitamin F (Polyen-Fettsäuren), β-Carotin (Provitamin A) und die lipidlöslichen Derivate (z.B. Ester) dieser Stoffe. Geeignet sind auch die lipidlöslichen Ester der Ascorbinsäure z.B. Stearylascorbat. Bevorzugt werden aber natürliche oder synthetische Tocopherole und deren lipidlöslichen Derivate in die erfindungsgemäßen Körperreinigungsmittel eingesetzt.

Geeignete Tocopherole sind z.B. die natürlichen Tocopherole und deren Gemische sowie synthetische Tocopherole. Geeignete Ester sind z.B. Tocopherolacetat, Tocopherolnicotinat, Tocopherolascorbat, Tocopherylretinoat, Tocopherylsuccinat, Tocopheryllinoleat oder Tocopherylbenzoat.

Polare Lipide im Sinne der Erfindung sind Fettstoffe mit einer oder zwei linearen C₁₂-C₂₂-Alkyl- oder Acylgruppen und einer hydrophilen Gruppe, deren Größe nicht ausreicht, um das Molekül wasserlöslich zu machen. Solche hydrophilen Gruppen sind z.B. die Hydroxylgruppe, eine Dihydroxyethylgruppe oder eine Polyhydroxyalkoxygruppe mit 3 - 6 C-Atomen und 2 - 5 Hydroxylgruppen. Solche polaren Lipide werden oft auch als "lipophile Coemulgatoren" bezeichnet. Geeignete Beispiele für solche Lipide sind z.B. Cetyl- und Stearylalkohol, 1,2-Dodecandiol, Glycerinmonocetylether, Glycerinmonostearat. Stearylmonoglucosid, Sorbitanmonopalmitat oder Methylglucosid-dioleat.

Weitere geeignete Lipide sind auch die Phospholipide (Lecithine) und die Sterine (z.B. Cholesterin und Pflanzensterine).

Lipid-Tensid-Mischmizellen oder lamellare Flüssigkristalle mit lipidlöslichen Wirkstoffen werden erhalten, wenn man Gemische aus dem Wirkstoff, einem polaren Lipid und einem wasserlöslichen Tensid als Dispergator mit Wasser auf eine Temperatur oberhalb des Schmelzpunktes des Lipids erwärmt und vermischt. Dabei wird das Gewichtsverhältnis von Lipid zu Dispergator so eingestellt, daß eine lamellare Flüssigkristalldispersion bzw. unterhalb der Schmelztemperatur eine lamellare Gelphase gebildet wird, die bei Verdünnung mit Wasser Lipid-Tensid-Mischzellen ausbilden kann. Die Viskosität dieser Gelphase kann durch Zusatz kurzkettiger C₁-C₄-Alkohole oder Glycole, z.B. von Ethanol oder Propylenglycol in den fließfähigen Bereich gesenkt werden. Das Gewichtsverhältnis von Lipid zu Dispergator beträgt bevorzugt 1:2 bis 2:1. 1. Das Gewichtsverhältnis von polarem Lipid zu lipidlöslichem Wirkstoff beträgt bevorzugt 10 : 0,2 bis 10 : 2.

Die Herstellung der erfindungsgemäßen Körperreinigungsmittel erfolgt bevorzugt in der Weise, daß man ein Konzentrat des in Lipid-Tensid-Mischmizellen oder Flüssigkristallen solubilisierten Wirkstoffs mit einem Gehalt von 1 - 10 Gew.-% des Wirkstoffs herstellt und mit dem wäßrigen Körperreinigungsmittel vermischt. Dabei kann es vorkommen, daß je nach dem Mengenverhältnis des zugemischten lamellaren Flüssigkristall-Konzentrats zur wäßrigen Tensidzubereitung des Körperreinigungsmittels ein transparentes, optisch isotropes Produkt (Mischmizellen) oder ein trübes Produkt mit lamellaren Emulsionströpfchen entsteht, deren Größe nicht oberhalb 500 nm liegen sollte. Bevorzugt sollte daher ein transparentes Produkt erhalten werden, das den Lipid-Vitamin-Komplex in Form von Mischmizellen enthält.

Ein weiterer Gegenstand der Erfindung ist die Verwendung wässriger Zubereitungen, die nach dem Verfahren gemäß Patentanspruch 1 erhältlich sind, zum Waschen und Reinigen der Haut und der Haare und zur Erhöhung der Aufnahme des Wirkstoffs in die Haut.

Die erfindungsgemäßen Körperreinigungsmittel eignen sich, um schon durch Anwendung der erfindungsgemäßen Körpmeinigungsmittel beim Duschen, Baden oder Händewaschen auch eine kosmetische oder dermatologische Behandlung der Haut zu erreichen. Für eine solche Verwendung ist es besonders bevorzugt, dass als polares Lipid ein linearer C₁₂-C₂₂-Fettalkohol, ein lineares C₁₂-C₂₂-Alkandiol, ein C₈-C₂₂-Fettsäurepartialester eines C₃-C₈-Polyols mit 2 - 6 Hydroxylgruppen oder ein C₈-C₂₂-Fettsäuremono-alkanolamid enthalten ist, und dass die polaren Lipide in einer Menge von 5 bis 50 Gewichtsteilen, bezogen auf einen Gewichtsteil des Wirkstoffs, enthalten sind.

Die folgenden Beispiele sollen den Erfindungsgegenstand näher erläutern:

### Beispiele

### 1. Herstellung der Wirkstoffe-Konzentrate

### 1.1 Solubilisation in Lipid-Tensid-/Flüssigkristall-Dispersion

| | **S1** | **S2** | **S3** |
|---|---|---|---|
| Vitamin E-acetat | 1,8 | 2,1 | 2,9 |
| Vitamin F (Linolsäure) | 3,6 | 3,4 | 4,8 |
| Glycerinmonooleat | 23,0 | 23,6 | 19,2 |
| Alkyl-(C₈-C₁₆)-glucosid | 28,1 | 31,2 | 25,4 |
| 1,2-Propylenglycol | 9,5 | - | 15,5 |
| Wasser | 34,0 | 39,7 | 32,2 |
| Aussehen | trüb | trüb | trüb |
| Polaris. Mikroskop | L | L | L |
| L = lamellar | | | |

Die Herstellung erfolgte durch Mischen der Lipide, des Dispergators und der Vitamine oberhalb der Schmelztemperatur des Lipids, hier bei 60°C, und Vermischen mit Wasser und ggf. Propylenglycol (zur Senkung der Viskosität der lamellaren Gelphase).

### 1.2 Mikroemulgierung (nicht erfindungsgemäß)

| | **M1** | **M2** |
|---|---|---|
| Vitamin E-acetat | 1,0 | 1,0 |
| Vitamin F (Linolsäure) | 2,0 | 2,0 |
| Cetylpalmitat | 30,0 | 30,0 |
| hydr. Rizinusöl | 2,5 | 2,5 |
| Glycerinmonopalmitat | - | 0,7 |
| Behenylalkohol + 10 EO | 10,0 | 9,3 |
| Wasser | 54,5 | 54,5 |
| Aussehen | weiß-bläulich | weiß-bläulich |
| Partikelgröße [nm] | 172* | 199* |

| | | |
|---|---|---|
| * (gemessen mittels Laserlichtstreuung (Zetasizer von Malvern) | | |

Die Herstellung erfolgte nach dem sog. PIT-(Phaseninversionstemperatur)-Verfahren. Dabei werden das Wachs (Cetylpalmitat), das hydr. Rizinusöl, die Emulgatoren (Glycerinmonopalmitat, Behenylalkoholoxethylat) und die öllöslichen Wirkstoffe mit dem Wasser auf eine Temperatur oberhalb der Phaseninversionstemperatur (hier auf 80°C) erwärmt, intensiv gemischt und unter Rühren auf Normaltemperatur (20°C) abgekühlt. Es entstehen bläulich schimmernde, sehr feinteilige Emulsionen, die Wachs-Nanopartikel einer Teilchengröße unter 200 nm enthalten.

### 2. Vitaminhaltige Tensidformulierungen

Es wurde eine Duschbadformulierung D1 der folgenden Zusammensetzung verwendet:

| | **D1** |
|---|---|
| Na-Laurylsulfat (1) | 10,0 Gew.-% |
| Kokosfettsäure-Eiweißkondensat (2) | 1,0 Gew.-% |
| Kokosamidopropyl-betain (3) | 1,0 Gew.-% |
| Alkyl-(C₁₀-C₁₆)-glucosid (4) | 4,0 Gew.-% |
| Glycerin-(EO)₇-fettsäureester (5) | 1,0 Gew.-% |
| Fettalkohol-(C₁₂-C₁₄) + 2 EO (6) | 0,5 Gew.-% |
| Na-Benzoat | 0,4 Gew.-% |
| p-Hydroxybenzoesäureester | 0,3 Gew.-% |
| Na-Lactat | 0,2 Gew.-% |
| Milchsäure | 0,6 Gew.-% |
| d-Panthenol | 0,2 Gew.-% |
| NaCl | 0,5 Gew.-% |
| Wasser | ad 100 |

Die Herstellung erfolgte durch Vermischen der Komponenten bei Raumtemperatur.

Die Mengenangaben in der Rezeptur beziehen sich auf 100 % Aktivsubstanz. Die eingesetzten Handelsprodukte (Henkel KGaA) sind :
(1) Texapon^{®} N 70
(2) Lamepon^{®} S
(3) Dehyton^{®} K
(4) Plantaren^{®} 1200 CSUP
(5) Cetiol^{®} HE
(6) Arlypon^{®} F

Es wurden die folgenden vitaminhaltigen Formulierungen damit hergestellt :

| | **1** | **2** | **3** | **V** |
|---|---|---|---|---|
| Duschbad D 1 | 94 % | 92,5 % | 95 % | 99,7 % |
| Konzentrat M1 | 6 % | - | - | - |
| Konzentrat S 1 | - | 7,5 % | - | - |
| Konzentrat S2 | - | - | 5 % | - |
| Vitamin E-acetat | - | - | - | 0,1 % |
| Vitamin F (Linolsäure) | - | - | - | 0,2 % |
| Aussehen | bläulich-weiß | trüb lamellar | klar isotrop | klar isotrop |
| Partikelgröße [nm] | 172* | 1250** | - | - |

Die Herstellung erfolgte durch Vermischen bei Raumtemperatur. Die Vergleichsrezeptur V wurde auf übliche Weise analog dem Duschbad D1 hergestellt, wobei die Wirkstoffe in den Tensiden solubilisiert wurden.

Je nach Menge der eingemischten Flüssigkristall-Solubilisate wurden trübe Produkte (S 1 ) mit lamellaren Kügelchen oder transparente Mischmizellen-Solubilisate (S2) erhalten.

Die Messung der Partikelgröße erfolgte bei Rezeptur 1 mittels Laserlichtstreuung (Zetasizer von Malvern) und bei Rezeptur 2 mittels Videomikroskopie und digitaler Teilchengrößenbestimmung (Optimetries).

### 3. Penetrationsstudien (Vitamin E-acetat)

Für die Rezepturen 1 und 3 sowie die Vergleichsrezeptur V mit konventionell eingearbeiteten Wirkstoffen wurden Penetrationsstudien am perfundierten Rindereuter, dem sogenannten BUS-Modell (bovine udder skin model) durchgeführt. Die Methodik ist z.B. in ALTEX 12, 4/95, Seiten 196 - 200 näher beschrieben.

15 Minuten nach dem Beginn der Perfusion wurden je 4 g der Formulierungen auf Hautareale von 100 cm² aufgetragen. Nach 2 Minuten Einwirkungszeit wurde das Reinigungsmittel mit 2 1 warmen (40°C) Wassers unter Zuhilfenahme eines Schwamms gründlich abgespült und das Hautareal mit einem Papiertuch getrocknet.

Dieser Reinigungsvorgang wurde dreimal wiederholt, um einen möglichen Akkumulationseffekt zu berücksichtigen. Danach wurden Tesa^{®}-Strip-Filmabrisse (Tesa-Typ 4204, Fa. Beiersdorf) von den oberen Lagen des Stratum Corneum genommen. Es wurden jeweils 10 Filmabrisse genommen, was insgesamt einer etwa 10 µm dicken Schicht des Stratum Corneum entspricht. Pro Tesa-Strip (1,9 x 10 cm²) wurden dabei jeweils 1,10 mg Hornzellen abgelöst (Standardabweichung ± 0,36 mg für n = 20).

In den Hautproben wurde das Vitamin-E-acetat analytisch bestimmt. Dazu wurden die Tesa-Abrisse extrahiert und im Extrakt das Vitamin E-acetat flüssigkeitschromatographisch isoliert und substanzspezifisch bestimmt. Die Quantifizierung wurde durch Standardkalibrierung und Vergleich mit Blindproben (unbehandelte Hautareale) erreicht.

Es wurden die folgenden Penetrationsergebnisse erhalten :

| | **1** | **3** | **V** | **Blindwert** |
|---|---|---|---|---|
| Tesa-Strip 1 - 3 (kumulativ) µg | 1,66 | 1,69 | 0,72 | < 0,04 |
| Tesa-Strip 1 - 10 (kumulativ) µg | 3,48 | 2,73 | 1,16 | < 0,13 |

## Patentansprüche

1. Verfahren zur Herstellung eines Körperreinigungsmittels in Form einer wässrigen Zubereitung mit einem Gehalt von wenigstens 5 Gew.-% wasserlöslicher ionischer oder nichtionischer Tenside und wenigstens 0.05 Gew.-% eines lipidlöslichen, wasserunlöslichen kosmetischen oder dermatologischen Wirkstoffes, der mit einem polaren Lipid in Lipid-Tensid-Mischmizellen oder Flüssigkristallen solubilisiert ist, wobei die Emulsionsteilchen einen Durchmesser von weniger als 500 nm aufweisen, **dadurch gekennzeichnet, dass**
a) ein Konzentrat des in Lipid-Tensid-Mischmizellen oder Flüssigkristallen solubilisierten lipidlöslichen, wasserunlöslichen kosmetischen oder dermatologisehen Wirkstoffs mit einem Gehalt von 1- 10 Gew.-% des Wirkstoffs hergestellt wird,
a.i) indem ein Gemisch aus dem Wirkstoff, einem polaren Lipid, das ausgewählt ist aus einem linearen C₁₂-C₂₂-Fettalkohol, einem linearen C₁₂-C₂₂-Alkandiol, einem C₈-C₂₂-Fettsäurepartialester eines C₃-C₈-Polyols mit 2 - 6 Hydroxylgruppen oder einem C₈-C₂₂-Fettsäuremonoalkanolamid eines C₂- oder C₃-Alkanolamins und einem wasserlöslichen Tensid als Dispergator, das ausgewählt ist aus Alkylglycosiden bzw. Alkyl-(oligo)-glucosiden, mit Wasser auf eine Temperatur oberhalb des Schmelzpunktes des Lipids erwärmt und vermischt wird, wobei das Gewichtsverhältnis von polarem Lipid zu lipidlöslichem Wirkstoff 10 : 0,2 bis 10 : 2 und das Gewichtsverhältnis von Lipid zu Dispergator 1 : 2 bis 2 : 1 beträgt, so dass eine lamellare Flüssigkristalldispersion bzw. unterhalb der Schmelztemperatur eine lamellare Gelphase gebildet wird, die bei Verdünnung mit Wasser Lipid-Tensid-Mischzellen ausbildet,
b) und mit einer wässrigen Tensidzubereitung vermischt wird.

2. Verfahren gemäß Anspruch 1, **dadurch gekennzeichnet, dass** das Körperreinigungsmittel 5- 30 Gew.-% wasserlöslicher Tenside und 0.1 - 1 Gew.-% eines lipidlöslichen Vitamins als Wirkstoff enthält.

3. Verfahren gemäß Anspruch 1, **dadurch gekennzeichnet, dass** als wasserlösliche Tenside anionische Sulfat- oder Sulfonal-Tenside, ampholytische, zwitterionische, nichtionische Tenside oder Gemische davon enthalten sind.

4. Verfahren gemäß einem der Ansprüche 1 bis 3, **dadurch gekennzeichnet, dass** als Wirkstoffe Vitamin A (Retinole), Vitamin E, Vitamin F (Polyen-Fettsäuren), β-Carotin (Provitamin A), natürliche oder synthetische Tocopherole und die lipidlöslichen Derivate dieser Stoffe oder die lipidlöslichen Ester der Ascorbinsäure enthalten sind.

5. Körperreinigungsmittel, erhältlich nach dem Verfahren gemäß einem der Ansprüche 1 bis 4.

6. Nichttherapeutische Verwendung eines körperreinigungsmittels gemäß Anspruch 5 zum Waschen und Reinigen der Haut oder der Haare und zur Erhöhung der Aufnahme des Wirkstoffs in die Haut.

## Claims

1. A method for preparing a body cleansing agent as an aqueous preparation with a water-soluble ionic or non-ionic surfactant content of at least 5% by weight and a water-insoluble, liposoluble, cosmetic or dermatological active ingredient content of at least 0.05% by weight, which ingredient is solubilized with a polar lipid in mixed lipid/surfactant micelles or liquid crystals, wherein the particles of the emulsion have a diameter less than 500 nm,
**characterized in that**,
a) a concentrate of the water-insoluble, liposoluble, cosmetic or dermatological active ingredient solubilized in mixed lipid/surfactant micelles or liquid crystals with an active ingredient content from 1 to 10% by weight is prepared,
a.i) by heating and mixing a mixture of the active ingredient, of a polar liquid, which is selected from a linear C₁₂-C₂₂ fatty alcohol, a linear C₁₂-C₂₂ alkanediol, a partial ester of a C₈-C₂₂ fatty acid and C₃-C₈ polyol with 2 to 6 hydroxyls, or a monoalkanolamide of a C₈-C₂₂ fatty acid and a C₂ or C₃ alkanolamine, and a water-soluble surfactant as dispersion agent, which is selected from alkylglycosides or alkyl-(oligo)-glucosides, with water, at a temperature above the melting point of the lipid, wherein the weight ratio of the polar lipid to the liposoluble active ingredient is 10:0.2 to 10:2 and the weight ratio of the lipid to the dispersion agent is 1:2 to 2:1, so as to form a lamellar liquid crystal dispersion or a lamellar gel phase below the melting point, which forms mixed lipid/surfactant micelles when diluted with water,
b) and mixed with an aqueous surfactant preparation.

2. The method according to claim 1, **characterized in that** the body cleansing agent contains from 5 to 30% by weight of water-soluble surfactants and from 0.1 to 1% by weight of a liposoluble vitamin as an active ingredient.

3. The method according to claim 1, **characterized in that** anionic sulfate or sulfonate surfactants, ampholytic, zwitterionic, non-ionic surfactants or mixtures thereof are contained as water-soluble surfactants.

4. The method according to any of claims 1 to 3, **characterized in that** vitamin A (retinols), vitamin E, vitamin F (polyene fatty acid), β-carotene (provitamin A), natural or synthetic tocopherols and liposoluble derivatives of these substances or liposoluble esters of ascorbic acid, are contained as active ingredients.

5. A body cleansing agent, which may be obtained from the method according to any of claims 1 to 4.

6. The non therapeutic use of a body cleansing agent according to claim 5, for washing and cleaning the skin or hair and for increasing penetration of the active ingredient into the skin.

## Revendications

1. Procédé de préparation d'un nettoyant corporel sous forme d'une préparation aqueuse ayant une teneur en tensioactifs ioniques ou non ioniques hydrosolubles d'au moins 5% en poids et en une substance active cosmétique ou dermatologique, insoluble dans l'eau, liposoluble, d'au moins 0,05% en poids, qui est solubilisée avec un lipide polaire dans des micelles mixtes lipides/tensio-actifs ou des cristaux liquides, les particules de l'émulsion présentant un diamètre inférieur à 500 nm,
**caractérisé en ce que**,
a) un concentré de la substance active cosmétique ou dermatologique, insoluble dans l'eau, liposoluble, solubilisée dans des micelles mixtes lipides/tensio-actifs ou des cristaux liquides, ayant une teneur de 1 à 10% en la substance active est préparé,
a.i) en chauffant et en mélangeant un mélange de la substance active, d'un liquide polaire, qui est choisi parmi un alcool gras linéaire en C₁₂ à C₂₂, un alcanediol linéaire en C₁₂ à C₂₂, un ester partiel d'acide gras en C₈ à C₂₂ et d'un polyol en C₃ à C₈, avec 2 à 6 groupes hydroxyle, ou un monoalcanolamide d'acide gras en C₈ à C₂₂ et d'une alcanolamine en C₂ ou en C₃, et d'un tensio-actif hydrosoluble en tant qu'agent dispersant, qui est choisi parmi les alkylglycosides ou les alkyl-(oligo)-glucosides, avec de l'eau, à une température supérieure au point de fusion du lipide, le rapport pondéral du lipide polaire à la substance active liposoluble étant de 10:0,2 à 10:2, et le rapport pondéral du lipide à l'agent dispersant étant de 1 :2 à 2 :1 de sorte à former une dispersion lamellaire de cristal liquide ou une phase de gel lamellaire au-dessous de la température de fusion, laquelle phase forme des micelles mixtes lipides/tensio-actifs lors d'une dilution avec de l'eau,
b) et il est mélangé à une préparation aqueuse de tensio-actifs.

2. Procédé selon la revendication 1, **caractérisé en ce que** le nettoyant corporel contient de 5 à 30% en poids de tensio-actifs hydrosolubles et de 0,1 à 1% en poids d'une vitamine liposoluble en tant que substance active.

3. Procédé selon la revendication 1, **caractérisé en ce que** des tensio-actifs sulfate ou sulfonate anioniques, des tensio-actifs ampholytiques, zwitterioniques, non ioniques ou des mélanges de ceux-ci sont contenus en tant que tensio-actifs hydrosolubles.

4. Procédé selon l'une quelconque des revendications 1 à 3, **caractérisé en ce que** de la vitamine A (rétinols), de la vitamine E, de la vitamine F (acide gras polyène), du β-carotène (provitamine A), des tocophérols naturels ou synthétiques et des dérivés liposolubles de ces substances ou des esters liposolubles de l'acide ascorbique, sont contenus en tant que substances actives.

5. Nettoyant corporel, pouvant être obtenu d'après le procédé selon l'une quelconque des revendications 1 à 4.

6. Utilisation non-thérapeutique d'un nettoyant corporel selon la revendication 5, pour laver et nettoyer la peau ou les cheveux, et pour augmenter la pénétration de la substance active dans la peau.
